(19) Europäisches Patentamt
European Patent Office
Office européen des brevets

(11) EP 1 844 755 A1

(12) **DEMANDE DE BREVET EUROPEEN**

(43) Date de publication:
**17.10.2007 Bulletin 2007/42**

(21) Numéro de dépôt: **07105525.5**

(22) Date de dépôt: **03.04.2007**

(51) Int Cl.:
***A61K 8/36*** *(2006.01)* ***A61K 8/40*** *(2006.01)*
***A61K 8/22*** *(2006.01)* ***A61Q 5/06*** *(2006.01)*
***A61Q 5/12*** *(2006.01)*

(84) Etats contractants désignés:
**AT BE BG CH CY CZ DE DK EE ES FI FR GB GR HU IE IS IT LI LT LU LV MC MT NL PL PT RO SE SI SK TR**
Etats d'extension désignés:
**AL BA HR MK YU**

(30) Priorité: **13.04.2006 FR 0603318**

(71) Demandeur: **L'Oréal**
**75008 Paris (FR)**

(72) Inventeurs:
- **Jegou, Gwenaëlle**
  **93190, LIVRY GARGAN (FR)**
- **Mougin, Nathalie**
  **75011, PARIS (FR)**

(74) Mandataire: **Fevrier, Murielle Françoise E.**
**L'OREAL - D.I.P.I.**
**25-29 Quai Aulagnier**
**92600 Asnières (FR)**

(54) **Composition cosmétique comprenant au moins un monomère dielectrophile et un amorceur radicalaire**

(57) La présente invention a pour objet de nouvelles compositions pour le traitement cosmétique de fibres kératiniques telles que les cheveux, comprenant dans un milieu cosmétiquement acceptable au moins un monomère diélectrophile et au moins un amorceur radicalaire. L'invention concerne également un procédé de traitement de fibres kératiniques à partir de cette composition ainsi que son utilisation pour traiter lesdites fibres.

La composition conforme à la présente invention permet lorsqu'elle est appliquée sur les fibres kératiniques telles que les cheveux permet d'obtenir un gainage présentant une résistance aux agents extérieurs améliorée.

EP 1 844 755 A1

## Description

La présente invention a pour objet de nouvelles compositions pour le traitement cosmétique de fibres kératiniques telles que les cheveux, comprenant dans un milieu cosmétiquement acceptable au moins un monomère diélectrophile et au moins un amorceur radicalaire. L'invention concerne également un procédé de traitement de fibres kératiniques à partir de cette composition ainsi que son utilisation pour traiter lesdites fibres.

Il existe de nombreux produits de coiffage permettant d'apporter du corps, de la masse ou du volume aux cheveux. Un inconvénient lié à ces produits, le plus souvent à base de polymères filmogènes, réside dans le fait que l'effet cosmétique disparaît dès le premier shampooing.

Il est envisageable d'accroître la rémanence du dépôt de polymères en effectuant directement une polymérisation radicalaire de certains monomères au niveau des cheveux comme décrit dans le brevet américain US 5362486. Une autre variante est de traiter les cheveux après réduction de la kératine des cheveux, par polymérisation radicalaire de monomères de type MMA (monoélectrophile) ou d'acide itaconique.

Toutefois, les traitements ainsi obtenus sont peu cosmétiques et on constate généralement une forte dégradation de la fibre. Les cheveux ainsi traités sont difficilement démêlables.

Par ailleurs il est connu de la demande de brevet FR 2 833 489 des compositions de traitement des cheveux à partir des compositions comprenant des monomères électrophiles capables de se polymériser par voie anionique directement sur le cheveu. Ces monomères après polymérisation permettent d'obtenir des cheveux parfaitement gainés. Cependant le gainage obtenu ne présente pas une résistance satisfaisante par rapport aux différentes agressions extérieures que peuvent subir les cheveux.

Il existe donc un besoin de trouver de nouvelles compositions cosmétiques permettant d'apporter du corps, de la masse et/ou du volume à la chevelure, et ce de façon durable, en particulier face aux diverses agressions que peuvent subir les cheveux telles que les shampooings, les frottements, la lumière, les intempéries, la sueur et les déformations permanentes. Ces propriétés sont particulièrement remarquables en ce qui concerne la résistance vis-à-vis des shampooings.

Ce but est atteint avec la présente invention qui a pour objet une composition, comprenant dans un milieu cosmétiquement acceptable au moins un monomère diélectrophile et au moins un amorceur radicalaire.

Une telle composition lorsqu'elle est appliquée sur les fibres kératiniques telles que les cheveux permet d'obtenir un gainage présentant une résistance aux agents extérieurs améliorée tout en conservant des cheveux parfaitement individualisés, pouvant être coiffés sans problème. Les propriétés de coiffant apportées à la fibre sont de plus rémanentes. En effet, il se forme ainsi à la surface des cheveux un gainage protecteur et rémanent aux diverses agressions que peuvent subir les cheveux telles que les shampooings, les frottements, la lumière, les intempéries, la sueur, le sébum et les déformations permanentes. Ces propriétés sont particulièrement remarquables en ce qui concerne la résistance de la coloration vis-à-vis des shampooings. Le film formé est hydrophobe et particulièrement résistant mécaniquement.

Le gainage ainsi obtenu est homogène, lisse et possède une excellente adhésion sur les cheveux.

Le polymère formé enrobe la fibre kératinique formant un gainage de ladite fibre lui donnant de la masse, du volume, de la brillance et de la douceur.

La présente invention a aussi pour objet un procédé de traitement des fibres kératiniques qui comprend l'application sur les matières kératiniques de la composition de l'invention ainsi que l'utilisation de la composition de l'invention pour le traitement desdites fibre telles que les cheveux.

Elle a également pour objet un kit comprenant d'une part la composition contenant au moins un monomère diélectrophile et d'autre part une deuxième composition qui contient au moins un amorceur radicalaire.

Le monomère diélectrophile utile à l'invention est le monomère de formule (I) :

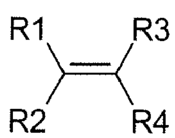

(A)

dans laquelle :

- R1 et R2 désignent chacun, indépendamment l'un de l'autre, un groupe peu ou non électro-attracteur, peu ou non inductif attracteur, tel que :

    - un atome d'hydrogène,

- un groupe hydrocarboné saturé ou non, linéaire, ramifié ou cyclique, comportant de préférence de 1 à 20, mieux encore de 1 à 10 atomes de carbone, et contenant éventuellement un ou plusieurs atomes d'azote, d'oxygène, de soufre, et éventuellement substitué par un ou plusieurs groupements choisis parmi -OR, -C(O)OR, -C(O)R, -SR, et les atomes d'halogène,
- un résidu polyorganosiloxane modifié ou non,
- un groupement polyoxyalkylène,

de préférence R1 et R2 représentent un atome d'hydrogène ; ou avantageusement R1 représente un atome d'hydrogène et R2 représente un groupement phényle éventuellement substitué par un atome d'halogène ;

- R3 et R4 désignent chacun, indépendamment l'un de l'autre, un groupe électro-attracteur, choisi de préférence parmi les groupements $-N(R)_3^+$, $-S(R)_2^+$-, $-NO_2$, $-SO_2R$, -C≡N, -C(O)OR, -C(O)SR, $-C(O)NR_2$, -F, -Cl, -Br, -I, -OR, -C(O)R, -SR les groupes alcényle linéaires ou ramifiés, les groupes alcynyle linéaires ou ramifiés, les groupements mono- ou polyfluoroalkyle en $C_1$-$C_4$, les groupements aryle tels que phényle, ou les groupements aryloxy tels que phénoxyloxy ;
- R, identique ou différent, désigne un atome d'hydrogène ou un groupe hydrocarboné saturé ou non, linéaire, ramifié ou cyclique, comportant de préférence de 1 à 20, mieux encore de 1 à 10 atomes de carbone, et contenant éventuellement un ou plusieurs atomes d'azote, d'oxygène, de soufre, et éventuellement substitué par un ou plusieurs groupements choisis parmi -OR', -C(O)OR', -C(O)R', -SH, - SR', -OH, les atomes d'halogène, ou un résidu de polymère pouvant être obtenu par polymérisation radicalaire, par polycondensation ou par ouverture de cycle, R' désignant un groupe alkyle en $C_1$-$C_{10}$ ;

étant entendu que (A) ne peut représenter le 2-cyanoacrylate de méthyle ou l'acide itaconique.

Par groupement électro-attracteur on entend un groupement électro-attracteur par effet inducteur ou inductif-attracteur (-I).

Plus spécifiquement par effet (-I) on entend tout groupement plus électronégatif que le carbone. On pourra se reporter à l'ouvrage PR Wells Prog. Phys. Org. Chem., 1968, Vol 6, pp 111.

Par groupement peu ou non électro-attracteur, on entend tout groupement dont l'électronégativité est inférieure ou égale à celle du carbone.

Lorsque le monomère est cyclique, les groupes éléctro-attracteurs sont de préférence exocyclique, c'est-à-dire qu'il ne fait pas partie intégrante de la structure cyclique du monomère.

Les groupements alcényle ou alcynyle ont de préférence 2 à 20 atomes de carbone, mieux encore de 2 à 10 atomes de carbone.

Comme groupe hydrocarboné saturé ou non, linéaire, ramifié ou cyclique, comportant de préférence de 1 à 20 atomes de carbone, on peut notamment citer les groupes alkyle, alcényle ou alcynyle linéaires ou ramifiés, tels que méthyle, éthyle, n-butyle, tert-butyle, iso-butyle, pentyle, hexyle, octyle, butényle ou butynyle ; les groupes cycloalkyle ou aromatiques.

Comme groupe hydrocarboné substitué, on peut citer par exemple les groupes hydroxyalkyle ou polyhalogénoalkyle.

A titre d'exemples de résidus polymères, on trouve les hydrocarbonépolyorganosiloxanes éventuellement modifiés.

On peut notamment citer les polyalkylsiloxanes tels que les polydiméthylsiloxanes, les polyarylsiloxanes tels que les polyphénylsiloxanes, les polyarylalkylsiloxanes tels que les polyméthylphénylsiloxanes.

Parmi les polyorganosiloxanes modifiés, on peut notamment citer les polydiméthylsiloxanes à groupements polyoxyalkylène et/ou siloxy et/ou silanol et/ou amine et/ou imine et/ou fluoroalkyle.

Parmi les résidus polymères, on peut rencontrer également les groupements polyoxyalkylène, et on peut notamment citer les groupements polyoxyéthylène et les groupements polyoxypropylène ou leurs copolymères ayant de préférence 1 à 200 motifs oxyalkylénés.

Parmi les groupements mono- ou polyfluoroalkyle, on peut notamment citer des groupements tels que $-(CH_2)_n-(CF_2)_m-CF_3$ ou $-(CH_2)_n-(CF_2)_m-CHF_2$ avec n=1 à 20 et m= 1 à 20.

Les substituants R1 à R4 peuvent éventuellement être substitués par un groupement ayant une activité cosmétique. Les activités cosmétiques particulièrement utilisées sont obtenues à partir de groupements à fonctions colorantes, antioxydantes, filtres UV et conditionnantes.

A titre d'exemples de groupement à fonction colorante, on peut notamment citer les groupements azoiques, quinoniques, méthiniques, cyanométhiniques et triarylméthane.

A titre d'exemples de groupement à fonction antioxydante, on peut notamment citer les groupements de type butylhydroxyanisole (BHA), butylhydroxytoluène (BHT) ou vitamine E.

A titre d'exemples de groupement à fonction filtre UV, on peut notamment citer les groupements de types benzophénones, cinnamates, benzoates, benzylidène-camphres et dibenzoylméthanes.

A titre d'exemples de groupement à fonction conditionnante, on peut notamment citer les groupements cationiques et de type esters gras. Parmi les monomères diélectrophiles répondant à la formule (A) on peut citer:

- les dérivés benzylidène malononitrile (1), le 2-(4-chlorobenzylidène)-malononitrile (2) le 2-cyano-3-phényl acrylate d'éthyle (3) le, 2-cyano-3-(4-chloro-phényl) acrylate d'éthyle (4) décrits dans Sayyah, J. Polymer Research, 2000, p97 ;

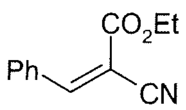
(1)

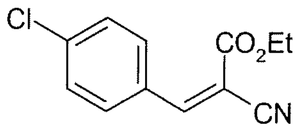
(2)

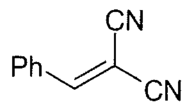
(3)

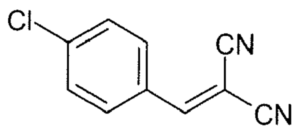
(4)

- les dérivés de méthylidènemalonates comme :

  ➢ Le 2-méthylène-malonate de diéthyle (5) par Hopff, Makromoleculare Chemie, 1961, p95, De Keyser, J. Pharm. Sci, 1991, p67 et Klemarczyk, Polymer, 1998, p173

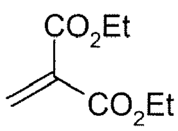
(5)

  ➢ Le alkyl-$C_1$-$C_8$ carboxyacrylate (5') décrit par Martinez JM. dans la demande de brevet WO9749436.

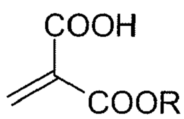
(5')

  ➢ le 2-éthoxycarbonylméthylèneoxycarbonyl acrylate d'éthyle (6) par Breton, Biomaterials, 1998, p271 et Couvreur, Pharmaceutical Research, 1994, p1270.

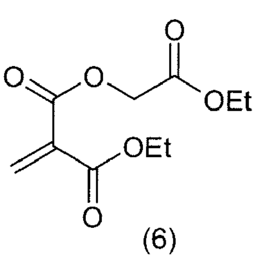
(6)

- Les dérivés α-(méthylsulfonyl)acrylates de méthyle (7) , α-(méthylsulfonyl)acrylates de d'éthyle (8), α-(tert-butylsulfonyl)acrylates de méthyle (9), α-(méthylsulfonyl)acrylates de tert-butyle (10), α-(tert-butylsulfonyl)acrylates de

tert-butyle (11) par Gipstein, J.Org.Chem, 1980, p1486 et les dérivés 1,1-bis-(méthylsulfonyl)éthylène (12), 1-acétyl-1-méthylsulfonyléthyléne (13), α-(méthylsulfonyl)vinyl sulfonate de methyle (14), α-methylsulfonylacrylonitrile (15) par Shearer, US patent US2748050.

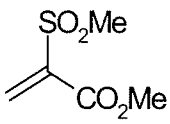
(7)

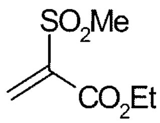
(8)

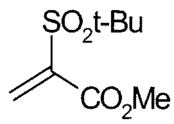
(9)

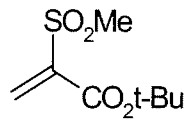
(10)

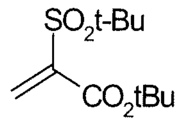
(11)

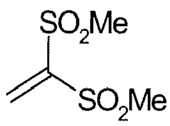
(12)

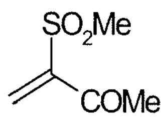
(13)

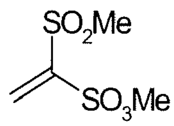
(14)

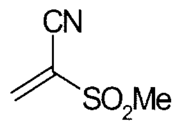
(15)

- Les dérivés itaconate et itaconimide autres que l'acide itaconique, tels que :

    ➢ l'itaconate de diméthyle (16) par Bachrach, European Polymer Journal, 1976, p563

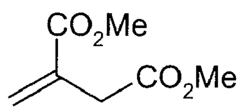
(16)

    ➢ N-butyl itaconimide (17), N-(4-tolyl) itaconimide (18), N-(2-éthylphényl) itaconimide (19), N-(2,6-diéthylphényl) itaconimide (20) par Wanatabe, J.Polymer Science : Part A :Polymer chemistry, 1994, p2073

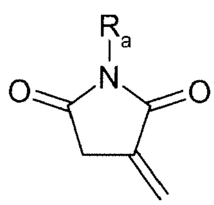

- $R_a$= Bu (21), 4-tolyl (22), 2-éthylphényl (23), 2,6-diéthyphényl (24)

Parmi les monomères précédemment cités, sont préférés les monomères de la famille des cyanoacrylates et leurs dérivés de formule (B):

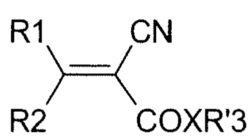
(B)

X désignant NH, S ou O,
R1 et R2 ayant les mêmes significations que précédemment, de préférence R1 et R2 représentant un atome d'hydrogène,
R'3 représentant R tel que défini à la formule (A),

De préférence, X désigne O.
De préférence R'3 représente un radical alkyle, linéaire ou ramifié, comprenant de 6 à 10 atomes de carbone ; ou alcényle comprenant 6 à 10 atomes de carbone.
A titre de composés de formule (B), on peut citer les monomères :

a) appartenant à la famille des 2-cyanoacrylates de polyfluoroalkyle tels que :

- l'ester 2,2,3,3-tétrafluoropropylique de l'acide 2-cyano-2-propénoïque de formule (D):

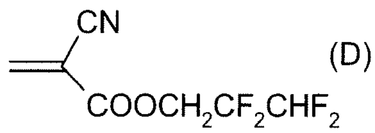

(D)

- ou encore l'ester 2,2,2-trifluoroéthylique de l'acide 2-cyano-2-propénoïque de formule (E):

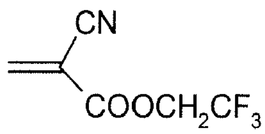

(E)

b) les 2-cyanoacrylates d'alkyle ou d'alcoxyalkyle de formule (C) :

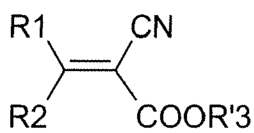

(C)

dans laquelle R'3 représente un radical alkyle en $C_1$-$C_{10}$, un radical alcényle en $C_2$-$C_{10}$ ou alcoxy($C_1$-$C_4$) alkyle ($C_1$-$C_{10}$) ;
on peut citer plus particulièrement le 2-cyanoacrylate d'éthyle, le 2-cyanoacrylate de n-propyle, le 2-cyanoacrylate d'isopropyle, le 2-cyanoacrylate de tert-butyle, le 2-cyanoacrylate de n-butyle, le 2-cyanoacrylate d'iso-butyle, le cyanoacrylate de 3-méthoxybutyle, le cyanoacrylate de n-décyle, le 2-cyanoacrylate d'hexyle, le 2-cyanoacrylate de 2-éthoxyéthyle, le 2-cyanoacrylate de 2-méthoxyéthyle, le 2-cyanoacrylate de 2-octyle, le 2-cyanoacrylate de 2-propoxyéthyle, le 2-cyanoacrylate de n-octyle, le cyanoacrylate d'isoamyle , 2-cyanoacrylate d'allyle et le 2-cyanoacrylate de méthoxypropyle ;
c) les dérivés benzylidène malonitrile de formule (1) et le 2-(4-chlorobenzylidène)-malonitrile de formule (2) et leurs mélanges

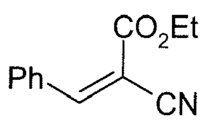

(1)

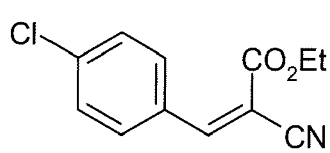

(2) .

Dans le cadre de l'invention, on préfère utiliser les monomères b) de formule (C). Selon un mode de réalisation préféré, le ou les monomères cyanoacrylates sont choisis parmi les cyanoacrylates d'alkyle en $C_6$-$C_{10}$ ou d'alcényle en $C_6$-$C_{10}$.

Les monomères particulièrement préférés sont les cyanoacrylates d'octyle de formule (F) et leurs mélanges :

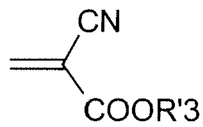

(F)

dans laquelle :

R'3= $-(CH_2)_7-CH_3$,
$-CH(CH_3)-(CH_2)_5-CH_3$,
$-CH_2-CH(C_2H_5)-(CH_2)_3-CH_3$,
$-(CH_2)_5-CH(CH_3)-CH_3$,
$-(CH_2)_4-CH(C_2H_5)-CH_3$.

Le monomère cyanoacrylate particulièrement préféré selon l'invention est l'octyl 2-cyanoacrylate, linéaire ou ramifié, vendu sous la référence commerciale RITELOK CDN 1064 par la société Chemence.

Les monomères utilisés conformément à l'invention peuvent être fixés de façon covalente sur des supports tels que des polymères, des oligomères ou des dendrimères. Le polymère ou l'oligomère peut être linéaire, ramifié, en peigne ou bloc. La répartition des monomères de l'invention sur la structure polymérique, oligomérique ou dendritique peut être statistique, en position terminale ou sous forme de blocs.

Les monomères cyanoacrylates de formule (B) selon la présente invention peuvent être synthétisés selon les méthodes connues décrites dans la technique. En particulier, les monomères cyanoacrylates peuvent être synthétisés selon l'enseignement des brevets US 3 527 224, US 3 591 767, US 3 667 472, US 3 995 641, US 4 035 334 et US 4 650 826.

Le monomère électrophile, et en particulier le monomère cyanoacrylate de formule (B), notamment l'octyl 2-cyanoacrylate peut être présent en une teneur allant de 0,1 et 99 % en poids, par rapport au poids total de la composition le comprenant.

Dans la composition de l'invention, la quantité de monomères cyanoacrylate est comprise entre 0,1 et 80 % en poids du poids total de la composition, de préférence entre 1 et 50 %.

Dans le cadre de l'invention on entend par polymérisation radicalaire toute polymérisation initiée par un générateur ou amorceur de radicaux.

L'amorceur radicalaire est soluble dans l'eau ou dans les solvants organiques.

Il peut s'agir d'amorceur de type peroxyde notamment de formule générale (I) ou (II), azoïque, persulfate, oxido réducteur ou leur mélanges :

$R_1$-O-O-$R_2$ (I)

$R_1$-O-O-$R_3$-O-O-$R_2$ (II)

dans lesquelles :

- ➢ $R_1$ et $R_2$, identiques ou différents, représente un atome d'hydrogène ou un groupement choisi parmi aryle éventuellement substitué, ($C_1$-$C_6$)alkyle linéaire ou ramifié, acyle R'C(O)- ou -C(O)R", et ester R'OC(O)- ou -(O)COR", avec R', R", identiques ou différents, représentant un groupement ($C_1$-$C_6$)alkyle linéaire ou ramifié, ou aryle éventuellement substitué ;
- ➢ $R_3$ représente un radical alkylène bivalent $-(CR_4R_5)_q-$ dans lequel $R_4$ et $R_5$, identiques ou différents, représentent un atome d'hydrogène, d'halogène ou un groupement ($C_1$-$C_6$)alkyle linéaire ou ramifié, et q représente un entier compris inclusivement entre 1 et 6.

Par radical « aryle éventuellement substitué» on entend un groupement mono ou polycyclique, condensé ou non, comprenant de 6 à 22 atomes de carbones, et dont au moins un cycle est aromatique ; préférentiellement le radical aryle est un phényle, biphényle, naphtyle, indényle, anthracényle, ou tétrahydronaphtyle ; ledit groupe étant éventuellement substitué par un radical choisi parmi :

- alkyle en $C_1$-$C_{16}$, de préférence en $C_1$-$C_8$ ;
- un atome d'halogène tel que chlore, fluor ou brome ;
- un groupement hydroxyle ;
- un radical alcoxy en $C_1$-$C_2$ ; un radical (poly)-hydroxyalcoxy en $C_2$-$C_4$ ;
- un radical amino éventuellement substitué par un ou deux radicaux alkyle, identiques ou différents, en $C_1$-$C_6$ ;
- un radical acylamino -$NR_6$-C(O)$R_7$ dans lequel le radical $R_6$ et $R_7$, identiques ou différents, représentent atome d'hydrogène, un radical alkyle en $C_1$-$C_4$ ;
- un radical carbamoyle $R_6R_7$N-C(O)- dans lequel les radicaux $R_6$ et $R_7$" identiques ou différents, représentent un atome d'hydrogène, un radical alkyle en $C_1$-$C_4$ ;
- un radical alkylsulfonylamino $R_6SO_2$-$NR_7$- dans lequel le radical $R_6$ et $R_7$, identiques ou différents, représentent un atome d'hydrogène, un radical alkyle en $C_1$-$C_4$ ;
- un radical aminosulfonyle $R_6R_7$N-$SO_2$-) dans lequel les radicaux $R_6$ et $R_7$, identiques ou différents, représentent un atome d'hydrogène, un radical alkyle en $C_1$-$C_4$ ;
- un radical carboxylique sous forme acide ou salifiée (de préférence avec un métal alcalin ou un ammonium, substitué ou non) ;
- un groupement cyano (CN) ; et
- un groupement polyhalogénoalkyle, tels que les trihalogénoalkyles en $C_1$-$C_6$, préférentiellement le trifluorométhyle.

Parmi les peroxydes de formule (I) on peut citer les hydropéroxides de formule $R_1$-O-O-H tels que le peroxyde de benzoyle, le peroxyde d'acétyle, le peroxyde de lauroyle, le peroxyde de décanoyle. On peut citer notamment les peroxydes commerciaux notamment vendus par Arkema sous les noms suivants :
La réactivité des générateurs de radicaux est souvent donnée par sa durée de demie vie à différentes températures ou par sa température de demi vie à différents temps.
La durée de demie vie à d'autres températures peut être calculée *via* la formule suivante :

$$t_{½} = (\ln 2)/kd$$

avec $kd = A \cdot e^{-(Ea/RT)}$,
• Ea = 123,80 kJ/mole,
• R = 8,3142 J/mole·K
• A = $1{,}62E^{+14}s^{-1}$
• et T en Kelvin (K) correspond à la température en degré Celsius (˚C) à laquelle est ajoutée 273,15

| | | Température de décomposition 10h ($t_{1/2}$) |
|---|---|---|
| Luperox® TBH70 X | Tert-Butyl hydroperoxide | 170-172 ˚C, benzène<br>164 ˚C, chlorobenzène |
| Luperox® TAH85 | Tert-Amyl hydroperoxide | |
| Luperox® CU80 | Cumyl hydroperoxide | 158 ˚C, benzène<br>140 ˚C, chlorobenzène |
| Luperox® 2,5-2,5 | 2,5-Dimethyl-2,5-di-(hydroperoxy) hexane | |
| Luperox® DH | Diisopropylbenzenemono hydroperoxide | |
| Luperox® PMHP | Paramenthane hydroperoxide | |

Parmi les peroxydes on peut également citer les dialkylpéroxides de formule (I) tels que le peroxyde de benzoyle dicumyle et les peroxydes commerciaux vendu notamment par Arkema sous les noms suivants :

| | | Température de décomposition 10h ($t_{1/2}$) |
|---|---|---|
| Luperox® 130 | 2,5-Dimethyl-2,5-di (tert-butyl-peroxy) hexyne-(3) | 131 ˚C, benzene |

(suite)

| | | Température de décomposition 10h ($t_{1/2}$) |
|---|---|---|
| Luperox® DI | Di-tert-butyl peroxide | 129 ˚C, benzène |
| Luperox® DTA | Di-tert-amyl peroxide | 123 ˚C, benzène |
| Luperox® 101 | 2,5-Dimethyl-2,5-di (tert-butylperoxy) hexane | 120 ˚C, benzène |
| Luperox®DC Luperox® DCSC | Dicumyl peroxide | 115˚C, benzène |

Parmi les peroxydes, de formule (I) on peut mentionner les diacylperoxydes de formule R'-C(O)-O-O-C(O)-R" tels que ceux vendus notamment par Arkema sous les noms suivants :

| | | Température décomposition 10h ($t_{1/2}$) |
|---|---|---|
| Luperox® A75 | Benzoyl peroxide | 70-73 ˚C, benzène |
| Luperox® LP | Lauroyl peroxide | 65 ˚C, benzène |
| Luperox® DEC | Decanoyl peroxide | 61 ˚C, benzène |
| Luperox® 219M75 | 3,5,5-Trimethylhexanoyl | |
| Luperox® 219EN50 | 3,5,5-Trimethylhexanoyl peroxide | |

Parmi les peroxydes de formule (I) on trouve les peroxyesters de formule R'-C(O)-O-O-$R_2$ tels que le peroxybenzoate de t-butyle; le peroxypivalate de t-butyle (LUPERSOL™ 1, Atochem), le peroxy-2-éthylhexanoate de t-butyle (TRIGONOX™ 21-C50, Akzo Chemicals, Inc.), et ceux vendus par la société Arkema sous les noms suivants :

| | | Température de décomposition 10h ($t_{1/2}$) |
|---|---|---|
| Luperox® P | Tert-Butyl peroxybenzoate | 104 ˚C, benzène |
| Luperox® 7M50 | Tert-Butyl peroxyacetate | 102 ˚C, benzène |
| Luperox® 270 | Tert-Butyl peroxy - 3,5,5-trimethylhexanoate | |
| Luperox® 570 | Tert-Amyl peroxy - 3,5,5-trimethylhexanoate | |
| Luperox® 80M75 | Tert-Butyl peroxyisobutyrate | 82 ˚C, benzène |
| Luperox® 26 | Tert-Butyl peroxy-2-ethylhexanoate | 58 ˚C, benzène |
| Luperox® 575 | Tert-Amyl peroxy-2-ethylhexanoate | 75 ˚C, benzène |
| Luperox® 11M75 | Tert-Butyl peroxypivalate | 58 ˚C, benzène |
| Luperox® 554M75 | Tert-Amyl peroxypivalate | 55 ˚C, benzène |
| Luperox® 10 | Tert-Butyl peroxyneodecanoate | 48 ˚C, benzène |
| Luperox® 546M75 | Tert-Amtyl peroxyneodecanoate | 46 ˚C, benzène |
| Luperox® 188M70 | Cumyl peroxyneodecanoate | 38 ˚C, benzène |
| Luperox® 610EN50 | 3-Hydroxy-1,1- dimethylbutyl peroxyneodecanoate | |

Parmi les peroxydes de formule (I) on trouve les monoperoxycarbonates de formule R'-O-C(O)-O-O-$R_2$ tels que ceux vendus notamment par Arkema sous les noms suivants :

| | | Température de décomposition 10h ($t_{1/2}$) |
|---|---|---|
| Luperox® TBICM75 | OO-tert-Butyl-O-isopropyl-mono-peroxycarbonate | 99°C, benzène |
| Luperox® TBEC | OO-tert-Butyl-O-(2-ethylhexyl) mono-peroxycarbonate | 100 °C, benzène |
| Luperox® TAEC | OO-tert-Amyl-O-(2-ethylhexyl) mono-peroxycarbonate | 117 °C, benzène |
| Luperox® JWEB50 | poly(tert-butyl peroxycarbonate) | 119 °C, benzène |

Parmi les peroxydes de formule (I) on trouve les peroxydicarbonates de formule R'-O-C(O)-O-O-C(O)-O-R" tels que le peroxydicarbonate de diacetyle, peroxydicarbonate de di(4-t-butylcyclohexyl) (PERKADOX™ 16S, AKZO Chemicals), peroxydicarbonate de di(2-ethylhexyl) et le peroxydicarbonate de di-(2-éthylhexyl) (Luperox® 223).

Parmi les peroxydes de formule (II) on trouve les peroxyketals de formule $R_1$-O-O-$CR_4R_5$-O-O-$R_2$ tels que ceux vendus notamment par Arkema sous les noms suivants :

| | | Température de décomposition 10h ($t_{1/2}$) |
|---|---|---|
| Luperox® 331 | di(tert-butylperoxy)cyclohexane | 97°C dodécane |
| Luperox® 233M50 | Ethyl 3,3-di (tert-butylperoxy) butyrate | 114°C dodécane |
| Luperox® 533M65 | Ethyl 3,3-di (tert-amylperoxy) butyrate | 112°C dodécane |
| Luperox® 230M50 | n-Butyl 4,4-di-(tert-butylperoxy) valerate | |
| Luperox® 220M50 | 2,2-Di (tert-butylperoxy) butane | |
| Luperox® 231 M50 | 1,1-Di (tert-butylperoxy) cyclohexane 3,3,5-tri méthylcyclohexane | 96°C dodécane |
| Luperox® 531M60 | 1,1-Di (tert-amylperoxy) cyclohexane | 93 °C dodécane |

Parmi les amorceurs solubles dans les solvants organiques, on peut citer les amorceurs azoïques suivants:

| | | Température de décomposition 10h ($t_{1/2}$) |
|---|---|---|
| 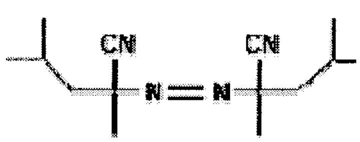 | Vazo® 52: 2'-azobis(2,4-dimethylvaleronitrile) de DuPont Chemicals | 52 °C, toluène |
| 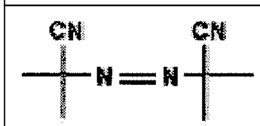 | Vazo® 64: 2,2'- Vazo® 64: 2,2' azobis (isobutyronitrile) de DuPont Chemicals | 64-65 °C, toluène |
| 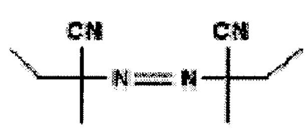 | Vazo® 67: 2,2'-azobis-2-methylbutyronitrile de DuPont Chemicals (VAZO™ 67 de DuPont Chemicals) Vazo® | 66 °C, chlorobenzène |
| 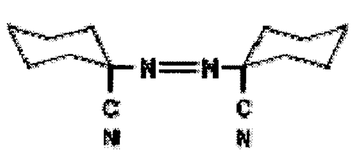 | 88: cyclohexanecarbonitrile de DuPont Chemicals, | °C, toluène 88 °C, |

2,2'-azobis(4-methoxy-2,4-dimethylvaleronitrile) (VAZO™ 33 de DuPont Chemicals) et 2,2'-azobis(methyl isobutyrate)

(V-601 de Wako).

Et parmi les amorceurs azoïques hydrosolubles, on peut citer:

| Code commercial | Formule de l'amorceur hydrosoluble | Température décomposition 10h ($t_{1/2}$) |
|---|---|---|
| VA-041 de Wako | 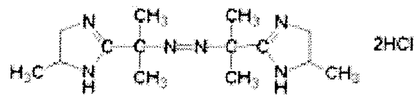 | 41 °C |
| VA-044 de Wako | 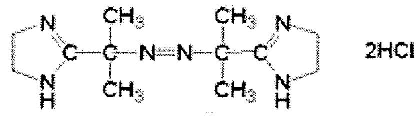 | 44°C |
| VA-046B de Wako | 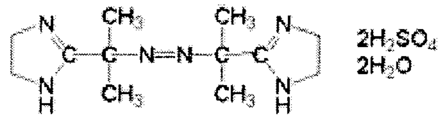 | 47°C |
| VA-057 de Wako | 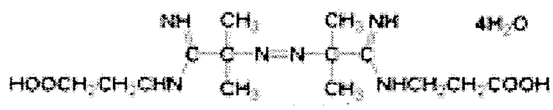 | 57°C |
| VA-058 de Wako | 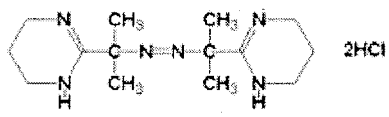 | 58°C |
| VA-060 de Wako | 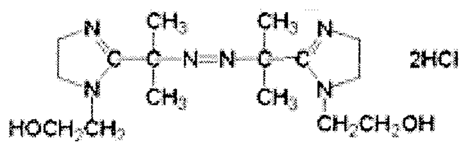 | 60°C |
| VA-061 de Wako | 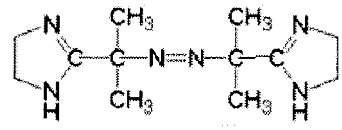 | 61°C |
| VA-080 de Wako | 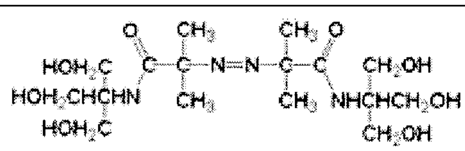 | 80°C |
| VA-085 de Wako | 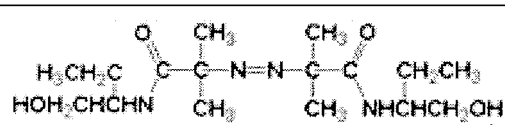 | 85°C |
| VA-085 de Wako | 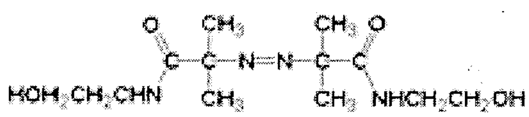 | 87°C |
| Vazo® 68 WSP de Du Pont Chemicals | 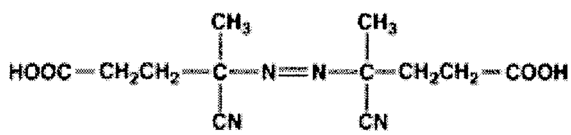 | |

(suite)

| Code commercial | Formule de l'amorceur hydrosoluble | Température décomposition 10h ($t_{1/2}$) |
|---|---|---|
| Vazo® et 56 WSW de DuPont ou V-50™ de Wako | 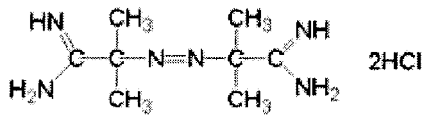 2,2′-azobis(2-amidinopropane)-dihydrochloride | 57°C |

L'amorceur est activé soit par voie photochimique, soit par voie thermique.

Parmi les amorceurs persulfate, on compte le persulfate de potassium (Température décomposition : 10h ($t_{1/2}$) = 60°C dans l'eau), sodium persulfate, et l'ammonium persulfate.

A titre d'amorceurs redox (d'oxidation-réduction), on peut utiliser les combinaisons de persulfate et d'agents réducteurs tels que le sodium métabisulfite ou le sodium bisulfite; mais également les systèmes à base de peroxydes et d'amines tertiaires (par exemple les couples: benzoyl peroxyde plus diméthylaniline); et les systèmes à bases d' hydroperoxydes et de métaux de transition tels que le mélange cumène hydroperoxyde plus cobalt naphthénate.

Parmi les photo-amorceurs capables d'être activés par une irradiation UV à des longueurs d'onde d'absorption comprises entre 250 nm à 450 nm, et de préférence supérieure à 351 nm on peut citer les benzoin éthers tels que les benzoin méthyl éther, benzoin isopropyl éther, les benzoin éthers substitués, les oxyde de arylphosphine, acétophénone subsituée telle que la 2,2-diméthoxy-2-phénylacétophénone, et alphacétol (alpha-hydroxycétones) substituée.

Les photoamorceurs disponibles commercialement sont par exemple les Irgacure™ 819 and Darocur™ 1173 (disponibles de Ciba-Geigy Corp., Hawthorne, N.Y.), Lucern TPO™ (disponibles de BASF, Parsippany, N.J.) and Irgacure™ 651, (2,2-diméthoxy-1,2-diphényl-1-éthanone), disponibles de Ciba-Geigy corporation.

Le choix de l'amorceur sera guidé en fonction de la cinétique de décomposition et donc de sa température de décomposition, la nature des agents de décomposition, de sa solubilité, etc.

Ainsi l'homme de l'art choisira l'amorceur en fonction de la température d'application souhaitée et de la durée de la réaction souhaitée.

Un mode de réalisation de l'invention concerne une composition contenant au moins un amorceur possèdant une température de décomposition 10h ($t_{1/2}$) comprise entre 45 °C et 180 °C. Préférentiellement comprise entre 45 °C et 120 °C.

L'amorceur radicalaire représente de 0.01 % à 40% en poids du monomère dilélectrophile de préférence de 0,1 % à 35 %. Plus préférentiellement de 1 % à 30 % en poids par rapport audit monomère.

La polymérisation peut éventuellement être assistée par un chauffage du monomère en présence de l'amorceur. La température appliquée variant entre 20°C et 100°C et de préférence entre 20°C et 90°C.

Le milieu des compositions de l'invention peut contenir, outre le ou les solvants organiques liquides, de l'eau. De préférence le milieu est anhydre c'est-à-dire contenant moins de 1% en poids d'eau par rapport au poids total de la composition.

Le milieu cosmétique de la composition de l'invention peut être utilisé homogène, se présenter sous forme d'une émulsion ou être encapsulé. La phase dispersée ou continue de l'émulsion peut alors être constituée par de l'eau, des alcools aliphatiques en $C_1$-$C_4$ ou leurs mélanges. Les capsules ou microcapsules contenant la composition de l'invention peuvent être dispersées dans un milieu anhydre tel que défini précédemment, de l'eau, des alcools aliphatiques en $C_1$-$C_4$ ou leurs mélanges.

Le milieu des compositions de l'invention peut contenir au moins un solvant organique liquide différent du monomère cyanoacrylate.

Par solvant organique, on entend une substance organique capable de dissoudre une autre substance sans la modifier chimiquement.

Les solvants organiques sont choisis parmi les composés liquides à la température de 25 °C et sous 105 Pa (760 mm de Hg).

Le solvant organique est par exemple choisi parmi les alcools aromatiques tels que l'alcool benzylique ; les alcools gras liquides , notamment en $C_{10}$-$C_{30}$; les polyols modifiés ou non tels que le glycérol, le glycol, le propylène glycol, le dipropylène glycol, le butylène glycol, le butyle diglycol ; les silicones volatiles telles que la cylopentasiloxane, la cyclohexasiloxane, les polydiméthylsiloxanes modifiées ou non par des fonctions alkyle et/ou amine et/ou imine et/ou fluoroalkyl et/ou carboxylique et/ou betaïne et/ou ammonium quaternaire, les polydiméthylsiloxanes modifiées liquides, les huiles minérales, organiques ou végétales, les alcanes et plus particulièrement les alcanes de $C_5$ à $C_{10}$ ; les acides gras liquides, et les esters gras liquides et plus particulièrement les benzoates ou les salicylates d'alcool gras liquides.

Le solvant organique est de préférence choisi parmi les huiles organiques ; les silicones telles que les silicones volatiles, les gommes ou huiles de silicones aminés ou non et leurs mélanges ; les huiles minérales ; les huiles végétales telles que les huiles d'olive, de ricin, de colza, de coprah, de germe de blé, d'amande douce, d'avocat, de macadamia, d'abricot, de carthame, de noix de bancoulier, de camélina, de tamanu, de citron ou encore des composés organiques tels que des alcanes en $C_5$-$C_{10}$, l'acétone, la méthyléthylcétone, les esters d'acides en $C_1$-$C_{20}$ liquides et d'alcools en $C_1$-$C_8$ tels que l'acétate de méthyle, l'acétate de butyle, l'acétate d'éthyle et le myristate d'isopropyle, le diméthoxyéthane, le diéthoxyéthane, les alcools gras liquides en $C_{10}$-$C_{30}$ tels que l'alcool oléique, les esters d'alcools gras en $C_{10}$-$C_{30}$ liquides tels que les benzoates d'alcool gras en $C_{10}$-$C_{30}$ et leurs mélanges ; l'huile de polybutène, l'isononanoate d'isononyle, le malate d'isostéaryle, le tétraisostéarate de pentaérythrityle, le trimélate de tridécyle, le mélange cyclopentasiloxane (14,7% en poids)/polydiméthylsiloxane dihydroxylé en positions $\alpha$ et $\omega$ (85,3% en poids), ou leurs mélanges.

Selon un mode de réalisation préféré, le solvant organique est constitué par une silicone ou un mélange de silicone tels que les polyalkylssiloxane et en particulier les polydiméthylsiloxanes liquides et les polydiméthylsiloxanes modifiées, liquides, la viscosité de la silicone et/ou du mélange de silicones à 25 °C est comprise entre 0.1 cst et 1 000 000 cst et plus préférentiellement entre 1 cst et 30 000 cst. La silicone peut également être une cycloalkylsiloxane.

On citera de préférence les huiles suivantes :

- le mélange de polydiméthylsiloxane alpha-omega-dihydroxylé /cyclopentadiméthylsiloxane (14,7/85,3) commercialisé par Dow Corning sous le nom de DC 1501 Fluid ;
- le mélange de polydiméthylsiloxane alpha-omega-dihydroxylé/polydiméthylsiloxane commercialisé par Dow Corning sous le nom de DC 1503 Fluid ;
- le mélange de diméthicone/cyclopentadiméthylsiloxane commercialisé par Dow Corning sous le nom de DC 1411 Fluid ou celui commercialisé par Bayer sous le nom SF1214 ;
- la cyclopentadiméthylsiloxane commercialisée par Dow Corning sous le nom de DC245 Fluid ;

Et les mélanges respectifs de ces huiles.

Le ou les solvants organiques de la composition représentent généralement de 0,01 à 99 %, de préférence de 50 à 99 % en poids par rapport au poids total de la composition.

La composition cosmétique selon l'invention peut comprendre en outre au moins un pigment.

L'utilisation d'un pigment dans la composition cosmétique conforme à l'invention permet d'obtenir des colorations visibles notamment sur des cheveux foncés puisque le pigment en surface masque la couleur naturelle de la fibre.

La composition conforme à l'invention présente ainsi l'avantage de conduire à des colorations qui présentent une bonne résistance aux diverses agressions que peuvent subir les cheveux, telles que les corps gras ou les shampooings.

De plus, la composition cosmétique selon l'invention permet de conduire à des colorations visibles et très chromatiques sur une fibre kératinique notamment foncée sans qu'il soit nécessaire d'éclaircir ou de décolorer les fibres kératiniques et, par conséquent, sans dégradation physique des fibres kératiniques.

Au sens de la présente invention, on entend par pigment toute entité organique et / ou minérale dont la solubilité dans l'eau est inférieure à 0,01 % à 20 °C, de préférence inférieure à 0,0001 %, et présentant une absorption entre 350 et 700 nm, de préférence une absorption avec un maximum.

Les pigments utilisés dans la composition selon l'invention peuvent être notamment choisis parmi les pigments organiques et/ ou minéraux connus de la technique, notamment ceux qui sont décrits dans l'encyclopédie de technologie chimique de Kirk-Othmer et dans l'encyclopédie de chimie industrielle de Ullmann.

Ces pigments peuvent se présenter sous forme de poudre ou de pâte pigmentaire. Ils peuvent être enrobés ou non enrobés.

Les pigments conformes à l'invention peuvent par exemple être choisis parmi les pigments blancs ou colorés, les laques, les pigments à effets spéciaux tels que les nacres ou les paillettes, et leurs mélanges.

A titre d'exemples de pigments minéraux blancs ou colorés, on peut citer le dioxyde de titane, traité ou non traité en surface, les oxydes de zirconium ou de cérium, les oxydes de fer ou de chrome, le violet de manganèse, le bleu outremer, l'hydrate de chrome et le bleu ferrique. Par exemple, les pigments minéraux suivants peuvent être utilisés : $Ta_2O_5$, $Ti_3O_5$, $Ti_2O_3$, TiO, $ZrO_2$ en mélange avec $TiO_2$, $ZrO_2$, $Nb_2O_5$ $CeO_2$, ZnS.

A titres d'exemples de pigments organiques blancs ou colorés, on peut citer les composés nitroso, nitro, azo, xanthène, quinoléine, anthraquinone, phtalocyanine, de type complexe métallique, isoindolinone, isoindoline, quinacridone, périnone, pérylène, dicétopyrrolopyrrole, thioindigo, dioxazine, triphénylméthane, quinophtalone.

En particulier, les pigments organiques blancs ou colorés peuvent être choisis parmi le carmin, le noir de carbone, le noir d'aniline, le jaune azo, la quinacridone, le bleu de phtalocyanine, le rouge sorgho, les pigments bleus codifiés dans le Color Index sous les références CI 42090, 69800, 69825, 73000, 74100, 74160, les pigments jaunes codifiés dans le Color Index sous les références CI 11680, 11710, 15985, 19140, 20040, 21100, 21108, 47000, 47005, les pigments verts codifiés dans le Color Index sous les références CI 61565, 61570, 74260, les pigments oranges

codifiés dans le Color Index sous les réfénces CI 11725, 15510, 45370, 71105, les pigments rouges codifiés dans le Color Index sous les références CI 12085, 12120, 12370, 12420, 12490, 14700, 15525, 15580, 15620, 15630, 15800, 15850, 15865, 15880, 17200, 26100, 45380, 45410, 58000, 73360, 73915, 75470, les pigments obtenus par polymérisation oxydante de dérivés indoliques, phénoliques tels qu'ils sont décrits dans le brevet FR 2 679 771.

On peut utiliser des pâtes pigmentaires de pigment organique telles que les produits vendus par la société HOECHST sous le nom :

- JAUNE COSMENYL IOG : Pigment YELLOW 3 (CI 11710) ;
- JAUNE COSMENYL G : Pigment YELLOW 1 (CI 11680) ;
- ORANGE COSMENYL GR : Pigment ORANGE 43 (CI 71105) ;
- ROUGE COSMENYL R : Pigment RED 4 (CI 12085) ;
- CARMIN COSMENYL FB : Pigment RED 5 (CI 12490) ;
- VIOLET COSMENYL RL : Pigment VIOLET 23 (CI 51319) ;
- BLEU COSMENYL A2R : Pigment BLUE 15.1 (CI 74160) ;
- VERT COSMENYL GG : Pigment GREEN 7 (CI 74260) ;
- NOIR COSMENYL R : Pigment BLACK 7 (CI 77266).

Les pigments conformes à l'invention peuvent aussi être sous forme de pigments composites tels qu'ils sont décrits dans le brevet EP 1 184 426. Ces pigments composites peuvent être composés notamment de particules comportant un noyau inorganique, au moins un liant assurant la fixation des pigments organiques sur le noyau, et au moins un pigment organique recouvrant au moins partiellement le noyau.

Par laque, on entend les colorants adsorbés sur des particules insolubles, l'ensemble ainsi obtenu restant insoluble lors de l'utilisation. Les substrats inorganiques sur lesquels sont adsorbés les colorants sont par exemple l'alumine, le borosilicate de calcium et de sodium ou le borosilicate de calcium et d'aluminium, et l'aluminium. Parmi les colorants organiques, on peut citer le carmin de cochenille.

A titre d'exemples de laques, on peut citer les produits connus sous les dénominations suivantes : D & C Red 21 (CI 45 380), D & C Orange 5 (CI 45 370), D & C Red 27 (CI 45 410), D & C Orange 10 (CI 45 425), D & C Red 3 (CI 45 430), D & C Red 7 (CI 15 850:1), D & C Red 4 (CI 15 510), D & C Red 33 (CI 17 200), D & C Yellow 5 (CI 19 140), D & C Yellow 6 (CI 15 985), D & C Green (CI 61 570), D & C Yellow 1 O (CI 77 002), D & C Green 3 (CI 42 053), D & C Blue 1 (CI 42 090).

Par pigments à effets spéciaux, on entend les pigments qui créent d'une manière générale une apparence colorée (caractérisée par une certaine nuance, une certaine vivacité et une certaine clarté) non uniforme et changeante en fonction des conditions d'observation (lumière, température, angles d'observation...). Ils s'opposent par-là même aux pigments blancs ou colorés qui procurent une teinte uniforme opaque, semi-transparente ou transparente classique.

A titre d'exemples de pigments à effets spéciaux, on peut citer les pigments nacrés blancs tels que le mica recouvert de titane, ou d'oxychlorure de bismuth, les pigments nacrés colorés tels que le mica recouvert de titane et d'oxydes de fer, le mica recouvert de titane et notamment de bleu ferrique ou d'oxyde de chrome, le mica recouvert de titane et d'un pigment organique tel que défini précédemment ainsi que les pigments nacrés à base d'oxychlorure de bismuth. A titre de pigments nacrés, on peut citer les nacres Cellini commercialisée par Engelhard (Mica-$TiO_2$-laque), Prestige commercialisée par Eckart (Mica-$TiO_2$), Prestige Bronze commercialisée par Eckart (Mica-$Fe_2O_3$), Colorona commercialisée par Merck (Mica-$TiO_2$-$Fe_2O_3$).

On peut également citer les pigments à effet interférentiel non fixés sur un substrat comme les cristaux liquides (Helicones HC de Wacker), les paillettes holographiques interférentielles (Geometric Pigments ou Spectra f/x de Spectratek). Les pigments à effets spéciaux comprennent aussi les pigments fluorescents, que ce soit les substances fluorescentes à la lumière du jour ou qui produisent une fluorescence ultraviolette, les pigments phosphorescents, les pigments photochromiques, les pigments thermochromiques et les quantum dots, commercialisés par exemple par la société Quantum Dots Corporation.

Les quantum dots sont des nanoparticules semi conductrices luminescentes capables d'émettre, sous excitation lumineuse, un rayonnement présentant une longueur d'onde comprise entre 400 nm et 700 nm. Ces nanoparticules sont connues de la littérature. En particulier, elles peuvent être fabriqués selon les procédés décrits par exemple dans le US 6 225 198 ou US 5 990 479, dans les publications qui y sont citées, ainsi que dans les publications suivantes : Dabboussi B.O. et al "(CdSe)ZnS core-shell quantum dots : synthesis and characterisation of a size series of highly luminescent nanocristallites" Journal of physical chemistry B, vol 101, 1997, pp 9463-9475. et Peng, Xiaogang et al, "Epitaxial Growth of highly Luminescent CdSe/CdS core/shell nanocrystals with photostability and electronic accessibility" Journal of the American Chemical Society, vol 119, N°30, pp 7019-7029.

La variété des pigments qui peuvent être utilisés dans la présente invention permet d'obtenir une riche palette de couleurs, ainsi que des effets optiques particuliers tels que des effets métalliques, interférentiels.

Selon un mode de réalisation particulier, les pigments sont des pigments colorés. On entend par pigment coloré

des pigments autres que les pigments blancs.

La taille du pigment utile dans le cadre de la présente invention est généralement comprise entre 10 nm et 200 μm, de préférence entre 20 nm et 80 μm, et plus préférentiellement entre 30 nm et 50 μm.

Les pigments peuvent être enrobés par des composés organiques ou minéraux.

L'agent organique avec lequel sont traités les pigments peut être déposé sur les pigments par évaporation de solvant, réaction chimique entre les molécules de l'agent de surface ou création d'une liaison covalente entre l'agent de surface et les pigments ou les charges.

Le traitement en surface au sens de la présente invention est tel qu'un pigment traité en surface conserve ses propriétés intrinsèques de pigment avant traitement.

Le traitement en surface peut ainsi être réalisé par exemple par réaction chimique d'un agent de surface avec la surface des pigments et création d'une liaison covalente entre l'agent de surface et les pigments. Cette méthode est notamment décrite dans le brevet US 4 578 266.

De préférence, on utilisera un agent organique lié aux pigments de manière covalente.

L'agent pour le traitement de surface peut représenter de 0,1 à 50 % en poids du poids total des pigments ou des charges traités en surface, de préférence de 0,5 à 30 % en poids, et encore plus préférentiellement de 1 à 10 % en poids.

De préférence, les traitements en surface des pigments sont choisis parmi les traitements suivants :

- un traitement PEG-Silicone comme le traitement de surface AQ commercialisé par LCW ;
- un traitement Chitosane comme le traitement de surface CTS commercialisé par LCW ;
- un traitement Triéthoxycaprylylsilane comme le traitement de surface AS commercialisé par LCW ;
- un traitement Méthicone comme le traitement de surface SI commercialisé par LCW ;
- un traitement Diméthicone comme le traitement de surface Covasil 3.05 commercialisé par LCW ;
- un traitement Diméthicone / Triméthylsiloxysilicate comme le traitement de surface Covasil 4.05 commercialisé par LCW ;
- un traitement Lauroyl Lysine comme le traitement de surface LL commercialisé par LCW ;
- un traitement Lauroyl Lysine Diméthicone comme le traitement de surface LL / SI commercialisé par LCW ;
- un traitement Myristate de Magnésium comme le traitement de surface MM commercialisé par LCW ;
- un traitement Dimyristate d'Aluminium comme le traitement de surface MI commercialisé par Miyoshi ;
- un traitement Perfluoropolyméthylisopropyl éther comme le traitement de surface FHC commercialisé par LCW ;
- un traitement Isostéaryl Sébacate comme le traitement de surface HS commercialisé par Miyoshi ;
- un traitement Disodium Stéaroyl Glutamate comme le traitement de surface NAI commercialisé par Miyoshi ;
- un traitement Diméthicone / Disodium Stéaroyl Glutamate comme le traitement de surface SA / NAI commercialisé par Miyoshi ;
- un traitement Phosphate de Perfluoroalkyle comme le traitement de surface PF commercialisé par Daito ;
- un traitement Copolymère acrylate / Diméthicone et Phosphate de Perfluoalkyle comme le traitement de surface FSA commercialisé par Daito ;
- un traitement Polyméthylhydrogène siloxane / Phosphate de Perfluoroalkyle comme le traitement de surface FS01 commercialisé par Daito ;
- un traitement Lauryl Lysine / Aluminium Tristéarate comme le traitement de surface LL-StAl commercialisé par Daito ;
- un traitement Octyltriéthylsilane comme le traitement de surface OTS commercialisé par Daito ;
- un traitement Octyltriéthylsilane / Phosphate de Perfluoroalkyle comme le traitement de surface FOTS commercialisé par Daito ;
- un traitement Copolymère Acrylate / Diméthicone comme le traitement de surface ASC commercialisé par Daito ;
- un traitement Isopropyl Titanium Triisostéarate comme le traitement de surface ITT commercialisé par Daito ;
- un traitement Cellulose Microcrystalline et Carboxyméthyl Cellulose comme le traitement de surface AC commercialisé par Daito ;
- un traitement Cellulose comme le traitement de surface C2 commercialisé par Daito ;
- un traitement copolymère Acrylate comme le traitement de surface APD commercialisé par Daito ;
- un traitement Phosphate de Perfluoroalkyle / Isopropyl Titanium Triisostéarate comme le traitement de surface PF + ITT commercialisé par Daito.

Le ou les pigments sont chacun généralement présents dans la composition conforme à l'invention dans des quantités généralement comprises entre 0,05 et 50 % du poids total de la composition, de préférence de 0,1 à 35 %.

Préférentiellement la composition de l'invention contient un pigment. Préférentiellement le pigment est un pigment à effets spéciaux de type Mica-$Fe_2O_3$ tel que le Prestige bronze.

La composition de l'invention peut contenir des inhibiteurs de polymérisation, et plus particulièrement des inhibiteurs de polymérisation anioniques et/ou radicalaires, ceci afin d'accroître la stabilité de la composition dans le

temps. De façon non limitative, on peut citer les inhibiteurs de polymérisation suivants : le dioxyde de soufre, l'oxyde nitrique, le trifluorure de bore, l'hydroquinone et ses dérivés tels que l'hydroquinone monéthyléther, la TBHQ, la benzoquinone et ses dérivés tels que la duroquinone, le catéchol et ses dérivés tels que le t-butyl catéchol et le méthoxycatéchol, l'anisole et ses dérivés tels que le méthoxyanisole ou l'hydroxyanisole, le pyrogallol et ses dérivés, le p-méthoxyphénol, l'hydroxybutyl toluène, les alkyl sulfates, les alkyl sulfites, les alkyl sulfones, les alkyl sulfoxydes, les alkyl sulfures, les mercaptans, le 3-sulfonène et leurs mélanges. Les groupements alkyle désignent de préférence des groupement ayant 1 à 6 atomes de carbone.

On peut aussi utiliser à titre d'inhibiteur les acides minéraux ou organiques.

Ainsi la composition cosmétique selon l'invention peut également comprendre au moins un acide minéral ou organique, ce dernier ayant un ou plusieurs groupements carboxyliques ou sulfoniques, présentant un pKa compris entre 0 et 6 tels que l'acide phosphorique, l'acide chlorhydrique, l'acide nitrique, l'acide benzène- ou toluène-sulfonique, l'acide sulfurique, l'acide carbonique, l'acide fluorhydrique, l'acide acétique, l'acide formique, l'acide propionique, l'acide benzoïque, les acides mono-, di- ou trichloroacétiques, l'acide salicylique et l'acide trifluoroacétique, l'acide octanoïque, l'acide heptanoïque et l'acide hexanoïque.

De préférence, l'acide acétique est utilisé.

La concentration en inhibiteur dans la composition cosmétique de l'invention peut être comprise entre 10 ppm et 30% en poids et plus préférentiellement entre 10 ppm et 15% en poids par rapport au poids total de la composition.

Le milieu cosmétique de la composition de l'invention peut également contenir un gélifiant/structurant spécifique comme les polyamides siliconés PSPA (DP100 et DP15) commercialisés par Dow Chemical, les KSG organiques polylauryldiméthylsiloxane et les KSG siliconés commercialisés par Shinetsu, le palmitate de dextrine et le stéarate d'inuline (gamme Rheopearl de Chiba Flour Milling) ; les acrylate à chaîne alkyle commercialisé par Landec, les copolymères éthylène octène commercialisé par Dupont de Nemours, le dibutyl lauryl glutamide commercialisé par Ajinomoto, le disorbène commercialisé par Roquette, les copolymères styrène acrylate Versagel M5960 et 5670 commercialisés par Penreco, les kratons dibloc et tribloc commercialisés par Kraton Polymers, l'acide hydroxystéariques, les cires de jojoba, les silicices pyrogénées commercialisées par Degussa, les cires de silicones commercialisés par Waker, le polyamide Uniclear commercialisé par Arizona Chemical, la bentone.

La composition de l'invention peut aussi se présenter sous forme d'une émulsion et/ou être encapsulé, les monomères électrophiles étant maintenus dans un milieu anhydre jusqu'au moment de l'utilisation. Lorsque la composition de l'invention est une émulsion, cette émulsion est par exemple constituée par une phase dispersée ou continue qui peut être constituée par de l'eau, des alcools aliphatiques en $C_1$-$C_4$ ou leurs mélanges et une phase organique anhydre comprenant le monomère. Dans le cas des capsules ou microcapsules, la capsule peut contenir le monomères dans un milieu anhydre et être dispersées dans un milieu anhydre tel que défini précédemment, de l'eau, des alcools aliphatiques en $C_1$-$C_4$ ou leurs mélanges.

La composition de l'invention peut aussi contenir un ou plusieurs polymères ne présentant pas de réactivité sur les monomères électrophiles et capable d'augmenter la viscosité de la composition. L'augmentation de la viscosité permet de réduire la vitesse de polymérisation des monomères électrophile. Pour ce faire, on peut ajouter à la composition de l'invention et de façon non exhaustive le polyméthylméthacrylate (PMMA) ou encore les copolymères à base de cyanoacrylate tels qu'ils sont décrits dans le brevet US 6,224,622.

La composition de l'invention peut aussi contenir des charges. Cela comprend à titre non limitatif des particules incolores ou blanches, minérales ou de synthèse, lamellaires ou non lamellaires. Elles peuvent être présentes à raison de 0 à 48 % en poids, par rapport au poids total de la composition, de préférence 0,01 à 30 % en poids, et mieux de 0,02 % à 20 % en poids. On peut notamment citer le talc, le stéarate de zinc, le mica, le kaolin, les poudres de polyamide (Nylon@) (Orgasol de chez Atochem), les poudres de polyéthylène, les poudres de polymères de tétrafluoroéthylène (TéflonB), l'amidon, le nitrure de bore, des microsphères polymériques telles que celles de chlorure de polyvinylidène/acrylonitrile comme l'Expancel (Nobel Industrie), de copolymères d'acide acrylique (PolytrapB de la société Dow Corning) et les microbilles de résine de silicone (TospearlsO de Toshiba, par exemple), les organopolysiloxanes élastomères.

Elle peut aussi contenir des poudres ou particules métalliques telles que des poudres ou particules d'aluminium, de zinc, de cuivre, etc.

La composition peut aussi contenir les actifs cosmétiques habituellement utilisés. On peut citer à titre non limitatif les agents réducteurs, les agents oxydants, les corps gras, les silicones, les agents épaississants, les adoucissants, les agents anti-mousse, les agents hydratants, les agents émollients, les agents alcalinisants, les élastomères, les plastifiants, les filtres solaires, les colorants directs ou d'oxydation, les argiles, les minéraux colloïdaux, les parfums, les peptisants, les conservateurs, les tensioactifs anioniques cationiques, amphotères, zwitterionqiues ou non-ionques, les polymères fixants ou non, les polymères conditionneurs, les protéines, les vitamines, etc.

Ces compositions peuvent se présenter sous des formes diverses, telles que des lotions, des sprays, des mousses et être appliquées sous forme de shampooing ou d'après-shampooing.

Dans le cas des sprays, la composition de l'invention peut contenir un propulseur. Le propulseur est constitué par les gaz comprimés ou liquéfiés usuellement employés pour la préparation de compositions aérosols. On emploiera

de manière préférentielle l'air, le gaz carbonique, l'azote comprimé ou encore un gaz soluble tel que le diméthyléther, les hydrocarbures halogénés (fluorés en particuliers) ou non et leurs mélanges.

Selon le procédé de l'invention, un mode de réalisation consiste à appliquer sur les fibres kératiniques telles que les cheveux, au moins un monomère diélectrophile et au moins un amorceur radicalaire.

L'amorceur et le monomère diélectrophile peuvent être appliqués de façon concomitante ou séparément. Dans ce dernier cas, l'amorceur peut être appliqué au préalable, ou inversement.

L'amorceur radicalaire peut être utilisé pur, en solution, sous forme d'une émulsion ou être encapsulé. Il peut aussi être ajouté à la composition au moment de l'emploi juste avant l'application sur les fibres kératiniques.

Préférentiellement la composition contenant au moins un monomère diélectrophile et au moins un amorceur radicalaire est appliquée sur les fibres kératiniques telles que les cheveux.

Si la composition contient un moyen de coloration tel qu'un pigment, un mode de réalisation de l'invention consiste en un procédé de de coloration par application sur les fibres kératiniques d'une composition contenant au moins un monomère diélectrophile et au moins un pigment.

Selon un autre mode de réalisation du procédé de l'invention, l'amorceur peut être appliqué dans une étape préalable ou ultérieure à l'application dudit monomère. Ces deux composants peuvent être appliqués purs ou en solution dans milieu cosmétiquement acceptable. Ils peuvent être solubles ou non dans le solvant. Un des solvants peut être un non solvant de l'amorceur radicalaire ou du monomère diélectrophile.

Selon un mode de réalisation particulier, il est possible de moduler la cinétique de polymérisation en humidifiant préalablement la fibre à l'aide d'une solution aqueuse dont le pH a été ajusté à l'aide d'une base, d'un acide ou d'un mélange acide/base. L'acide et/ou la base peuvent être inorganique ou organique.

Le procédé de l'invention peut comprendre des étapes additionnels intermédiaires ou finales telles que l'application d'un produit cosmétique, une étape de rinçage, une étape de séchage. Le séchage peut être effectué au casque, au sèche cheveux et/ou au fer à lisser. En particulier, l'application des compositions conformes à l'invention peut être suivie d'un rinçage.

Il est aussi possible de réaliser des applications multiples de la composition de l'invention afin d'obtenir une superposition de couches pour atteindre des propriétés spécifiques du dépôt en termes de nature chimique, résistance mécanique, épaisseur, aspect, toucher.

Afin d'améliorer entre autre l'adhésion des monomères diélectrophiles polymérisés *in situ,* la fibre peut être prétraitée avec tous types de polymères.

Pour moduler la cinétique de polymérisation anionique, on peut également augmenter la nucléophilie de la fibre par transformation chimique des fibres kératiniques. A titre d'exemple, on peut citer la réduction des ponts disulfures composant en partie la kératine en thiols avant application de la composition de l'invention. De façon non exhaustive, on peut citer comme réducteurs des ponts di-sulfure composant en partie la kératine, les composés suivants: thiosulfate de sodium anhydre, métabisulfite de sodium en poudre, thiourée, sulfite d'ammonium, acide thioglycolique, acide thiolactique, thiolactate d'ammonium, mono-thioglycolate de glycérol, thioglycolate d'ammonium, thioglycérol, acide 2,5-dihydroxybenzoique, di-thioglycolate de diammonium, thioglycolate de strontium, thioglycolate de calcium, formosulfoxylate de zinc, thioglycolate d'isooctyle, dl-cystéine, thioglycolate de monoéthanolamine.

L'application de la composition de l'invention peut aussi être précédée d'un traitement capillaire comme une coloration directe ou d'oxydation.

Selon la présente invention, les monomères diélectrophiles sont de préférence choisis parmi les monomères capables de polymériser sur les fibres kératiniques dans des conditions cosmétiquement acceptables.

En particulier, la polymérisation du monomère diélectrophile s'effectue de préférence à une température comprise entre 20 et 100°C, de préférence entre 20 et 90°C, ce qui n'empêche pas de terminer l'application par un séchage au casque, un brushing ou passage au fer plat ou à friser.

L'invention a aussi pour objet un kit de coloration comprenant une première composition qui contient le ou les monomères diélectrophiles et une deuxième composition qui contient le ou les amorceurs de radicaux.

Les exemples suivants non limitatifs permettent d'illustrer l'invention sans en limiter sa portée.

## EXEMPLES

Après avoir traitées les mèches avec la composition de l'invention, celles-ci sont enfermées dans du papier aluminium, et chauffées sur plaque chauffante 1 heure 72°C. Après avoir retiré le papier aluminium, les mèches sont séchées au casque à 40°C pendant 15 minutes. Les mèches sont ensuite rincées par l'application d'un shampooing doux. Cette étape représente l'essai initial ($t_0$).

Les mèches colorées sont ensuite soumises à 6 shampooings selon un cycle qui comprend le mouillage des mèches à l'eau, le lavage aux shampooings, un rinçage à l'eau suivi d'un séchage.

La couleur des mèches est alors évaluée sensoriellement à to et après 6 lavages aux shampooings.

Les mesures d'intensité lumineuse émise par les mèches sont réalisées avec un colorimètre MINOLTA (réf

CR 200).

La couleur des mèches a été évaluée à $t_0$ dans le système L*a*b*. Dans le système L* a* b*, L* représente l'intensité de la couleur, a* indique l'axe de couleur vert/rouge et b* l'axe de couleur bleu/jaune. Plus la valeur de L est faible, plus la couleur est foncée ou très intense. Plus la valeur de a* est élevée plus la nuance est rouge et plus la valeur de b* est élevée plus la nuance est bleue.

La variation de la couleur entre la mèche témoin constituée de cheveux blancs à 90% et la mèche traitée avec les compositions ci-dessous a été mesurée par ΔE selon l'équation ci dessous à partir des valeurs de L0*a0*b0* des mèches témoin et des valeurs de L*a*b* des mèches traitées avec la composition de l'invention.

$$\Delta E = \sqrt{(L^* - L_o{}^*)^2 + (a^* - a_o{}^*)^2 + (b^* - b_o{}^*)^2}$$

Plus la valeur de ΔE est importante, plus la différence de couleur entre la mèche témoin et la mèche traitée est élevé et donc plus le traitement est efficace.

Les résultats sont reportés dans les tableaux de résultats.

La saturation des mèches (SAT) a également été mesurée. Cette mesure correspond au rapport de la lumière colorée émise sur la lumière blanche réfléchie. SAT rend compte de la quantité de colorant présente sur la mèche. Plus sa valeur est élevée, plus le traitement est efficace.

**Exempte 1 :**

Mode d'application :

*L'amorceur se trouve dans la composition avec le monomère diélectrophile, et ladite composition est appliquée sur mèches sèches.*
On applique sur les mèches de cheveux 0,5 g de la composition ci-dessous.

Composition :

| | |
|---|---|
| octyl 2-cyanoacrylate | 0,5 g |
| Amorceur* | 0,1 g |
| Prestige bronze Eckart (Mica-$Fe_2O_3$) | 0,5 g |
| polydiméthylsiloxane α-ω-dihydroxylé /cyclopentadiméthylsiloxane (14,7/85,3) Dow Corning DC 1501 Fluid | 4,5 g |
| acide acétique | 12 µl |

* Les amorceurs testés sont les suivants:

1- persulfate de potassium, Aldrich
2- persulfate d'ammonium, Aldrich
3- V50 (2,2' azo bis (2-amidinopropane) dihydrochloride) : Interchim réf. 759356
4- 4,4' Azo bis (acide 4 -Cyanovalérique), Aldrich
5- persulfate de sodium : commercialisé par Aldrich

Résultats :

| Amorceur | ΔE | SAT |
|---|---|---|
| 1 | 10.70 | 25.18 |
| 2 | 16.19 | 31.44 |
| 3 | 5.12 | 28.86 |
| 4 | 2.47 | 31.77 |

(suite)

| Amorceur | ΔE | SAT |
|---|---|---|
| 5 | 4.71 | 27.57 |

Les mèches sont homogènes, d'un toucher délié.
La tenue après 6 shampooings des mèches traitées 1 à 5, est visuellement très satisfaisante, la couleur n'étant quasiment pas affectée.

**Exemple 2**

Mode d'application :

*L'amorceur se trouve dans la composition avec le monomère diélectrophile, et ladite composition est appliquée sur les mèches de cheveux immergées 3 minutes dans un tampon à pH3 puis essorées.*
On applique sur les mèches de cheveux 0,5 g de la composition de l'exemple 1.

Résultats :

| Amorceur | ΔE | SAT |
|---|---|---|
| 1 | 1,91 | 28,05 |
| 2 | 4,45 | 27,39 |
| 3 | 5,42 | 28,25 |
| 4 | 11,39 | 17,38 |
| 5 | 0,54 | 27.83 |

Les mèches sont homogènes, d'un toucher délié.
De la même façon, la tenue après 6 shampooings des mèches traitées 1 à 5, est visuellement très satisfaisante, la couleur n'étant quasiment pas affectée.

**Revendications**

1. Composition cosmétique comprenant au moins un monomère diélectrophile de formule (A) et au moins un amorceur radicalaire :

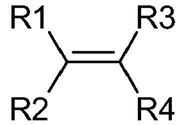 (A)

dans laquelle :

- R1 et R2 désignent chacun, indépendamment l'un de l'autre, un groupe peu ou non électro-attracteur ; et
- R3 et R4 désignent chacun, indépendamment l'un de l'autre, un groupe électro-attracteur,

étant entendu que (A) ne peut représenter le 2-cyanoacrylate de méthyle ou l'acide itaconique.

2. Composition selon la revendication précédente dans laquelle les monomères de formule (A) sont tels que :

- R1 et R2, identiques ou différents, représentent i) un atome d'hydrogène ; ii) un groupe hydrocarboné saturé ou non, linéaire, ramifié ou cyclique, comportant de préférence de 1 à 20, mieux encore de 1 à 10 atomes de carbone, et contenant éventuellement un ou plusieurs atomes d'azote, d'oxygène, de soufre, et éventuellement

substitué par un ou plusieurs groupements choisis parmi -OR, -C(O)OR, -C(O)R, -SR, et les atomes d'halogène ; iii) un résidu polyorganosiloxane modifié ou non ; iv) un groupement polyoxyalkylène ;

• R3 et R4, identiques ou différents, représentent un groupe choisi de parmi les groupements $-N(R)_3^+$, $-S(R)_2^+$-, $-NO_2$, $-SO_2R$ -C≡N, -C(O)OR, -C(O)SR, $-C(O)NR_2$, -F, -Cl, -Br, -I, -OR, -C(O)R, -SR, les groupes alcényle linéaires ou ramifiés, les groupes alcynyle linéaires ou ramifiés, les groupements mono- ou polyfluoroalkyle en $C_1$-$C_4$, les groupements aryle tels que phényle, ou les groupements aryloxy tels que phénoxyloxy ;

• R, identique ou différent, désigne un atome d'hydrogène ou une groupe hydrocarboné saturé ou non, linéaire, ramifié ou cyclique, comportant de préférence de 1 à 20, mieux encore de 1 à 10 atomes de carbone, et contenant éventuellement un ou plusieurs atomes d'azote, d'oxygène, de soufre, et éventuellement substitué par un ou plusieurs groupements choisis parmi -OR', -C(O)OR', - C(O)R', -SH, -SR', -OH, les atomes d'halogène, ou un résidu de polymère pouvant être obtenu par polymérisation radicalaire, par polycondensation ou par ouverture de cycle, R' désignant un groupe alkyle en $C_1$-$C_{10}$.

3. Composition selon les revendications précédentes dans laquelle le monomère diélectrophile est un monomère cyanoacrylate de formule (B) :

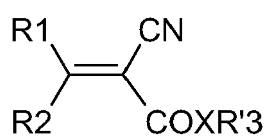

(B)

X désignant NH, S ou O,
$R'_3$ étant choisi parmi un atome et un groupe de R,
R, R1 et R2 étant tel que défini à la revendication 3.

4. Composition selon l'une quelconque des revendications précédente dans laquelle le monomère diélectrophile est un cyanoacrylate correspondant à la formule (C) :

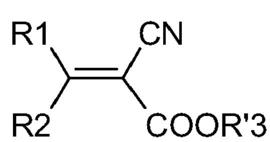

(C)

dans laquelle R'3 représente un radical alkyle en $C_1$-$C_{10}$, un radical alcényle en $C_2$-$C_{10}$ ou alcoxy($C_1$-$C_4$) alkyle ($C_1$-$C_{10}$) ; et R1 et R2 sont tels que définis dans la revendication 3.

5. Composition selon l'une quelconque des revendications précédente dans laquelle le monomère diélectrophile de formule (A) est tel que R1 et R2, identiques ou différents, sont choisis parmi l'atome d'hydrogène et un groupement phényle éventuellement substitué.

6. Composition selon l'une quelconque des revendications précédentes, dans laquelle le monomère diélectrophile est un cyanoacrylate d'alkyle de formule (F) :

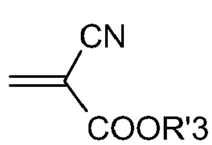

(F)

dans laquelle :

R'3 = $-(CH_2)_7-CH_3$,
$-CH(CH_3)-(CH_2)_5-CH_3$,

$-CH_2-CH(C_2H_5)-(CH_2)_3-CH_3$,
$-(CH_2)_5-CH(CH_3)-CH_3$,
$-(CH_2)_4-CH(C_2H_5)-CH_3$.

**7.** Composition selon l'une quelconque des revendications 1 à 5 dans laquelle le monomère diélectrophile est choisi parmi (1) et (2)

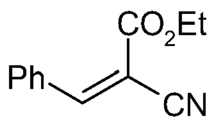

(1)

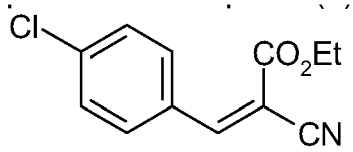

(2)

.

**8.** Composition selon l'une quelconque des revendications précédentes dans laquelle la quantité de monomères électrophile est comprise entre 0,1 et 80 % en poids du poids total de la composition.

**9.** Composition selon l'une quelconque des revendications précédentes, dans laquelle le ou les amorceurs radicalaires sont choisis parmi les peroxydes, les azoïques, les persulfates et les oxido réducteurs ; ou leur mélanges.

**10.** Composition selon la revendication précédente, dans laquelle le ou les amorceurs radicalaires peroxydes sont de formules générale (I) ou (II), ou leur mélange:

$R_1$-O-O-$R_2$ (I)

$R_1$-O-O-$R_3$-O-O-$R_2$ (II)

dans lesquelles :

➢ $R_1$ et $R_2$, identiques ou différents, représente un atome d'hydrogène ou un groupement choisi parmi aryle éventuellement substitué, (Ci-$C_6$)alkyle linéaire ou ramifié, acyle R'C(O)- ou -C(O)R", et ester R'OC(O)- ou -(O)COR", avec R', R", identiques ou différents, représentant un groupement ($C_1$-$C_6$)alkyle linéaire ou ramifié, ou aryle éventuellement substitué ;
➢ $R_3$ représente un radical alkylène bivalent -$(CR_4R_5)_q$- dans lequel $R_4$ et $R_5$, identiques ou différents, représentent un atome d'hydrogène, d'halogène ou un groupement ($C_1$-$C_6$)alkyle linéaire ou ramifié, et q représente un entier compris inclusivement entre 1 et 6.

**11.** Composition selon une quelconque des revendications précédentes dans laquelle l'amorceur représente de 0,01 à 40 % en poids du monomère diélectrophile.

**12.** Composition selon l'une quelconque des revendications précédentes dans laquelle le milieu contient un inhibiteur de polymérisation.

**13.** Composition selon l'une quelconque des revendications précédentes dans laquelle le milieu au moins un solvant organique.

**14.** Composition selon l'une quelconque des revendications précédentes dans laquelle le milieu contient un pigment.

**15.** Procédé de traitement des matières kératiniques **caractérisé par le fait que** l'on applique sur les fibres kératiniques notamment les cheveux, la composition telle que définie à l'une quelconque des revendications 1 à 14.

**16.** Kit de coloration comprenant un premier compartiment contenant une composition qui contient au moins un monomère diélectrophile tel que défini aux revendications 1 à 8, et un autre compartiment comprenant une deuxième composition qui contient au moins un amorceur radicalaire.

**17.** Utilisation d'une composition telle que définie aux revendications 1 à 13 pour le traitement des fibres kératiniques telles que les cheveux, permettant d'apporter du corps, de la masse et/ou du volume à la chevelure de façon durable.

**18.** Utilisation d'une composition telle que définie aux revendications 14 pour la coloration des fibres kératiniques telles que les cheveux.

Office européen des brevets

RAPPORT DE RECHERCHE EUROPEENNE

Numéro de la demande
EP 07 10 5525

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| X | US 3 634 022 A (ROBBINS ET AL.) 11 janvier 1972 (1972-01-11) | 1,2, 8-11,13, 15-18 | INV. A61K8/36 A61K8/40 A61K8/22 A61Q5/06 A61Q5/12 |
| Y | * le document en entier * | 3-7,12, 14 | |
| D,Y | FR 2 833 489 A (L'ORÉAL) 20 juin 2003 (2003-06-20) * page 1 - page 5; revendications 12,15; exemple 1 * | 3-7,12, 14 | |
| | | | **DOMAINES TECHNIQUES RECHERCHES (IPC)** |
| | | | A61K A61Q |

Le présent rapport a été établi pour toutes les revendications

| Lieu de la recherche | Date d'achèvement de la recherche | Examinateur |
|---|---|---|
| Berlin | 29 juin 2007 | ALVAREZ ALVAREZ, C |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
& : membre de la même famille, document correspondant

3

EPO FORM 1503 03.82 (P04C02)

## ANNEXE AU RAPPORT DE RECHERCHE EUROPEENNE RELATIF A LA DEMANDE DE BREVET EUROPEEN NO. EP 07 10 5525

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

29-06-2007

| Document brevet cité au rapport de recherche | Date de publication | Membre(s) de la famille de brevet(s) | Date de publication |
|---|---|---|---|
| US 3634022 A | 11-01-1972 | BE 751185 A1 | 03-11-1970 |
| | | CA 958336 A1 | 26-11-1974 |
| | | CH 547908 A | 11-04-1974 |
| | | DE 2025452 A1 | 28-01-1971 |
| | | DK 129023 B | 12-08-1974 |
| | | ES 379573 A1 | 16-10-1972 |
| | | FR 2043767 A5 | 19-02-1971 |
| | | GB 1321331 A | 27-06-1973 |
| | | IT 1013005 B | 30-03-1977 |
| | | NL 7007859 A | 01-12-1970 |
| | | NO 134184 B | 24-05-1976 |
| | | PH 11259 A | 02-11-1977 |
| | | ZA 7002931 A | 29-12-1971 |
| FR 2833489 A | 20-06-2003 | AU 2002366749 A1 | 09-07-2003 |
| | | BR 0215128 A | 03-11-2004 |
| | | EP 1455738 A2 | 15-09-2004 |
| | | WO 03053380 A2 | 03-07-2003 |
| | | JP 2005513087 T | 12-05-2005 |
| | | MX PA04005821 A | 10-09-2004 |
| | | US 2003175229 A1 | 18-09-2003 |

EPO FORM P0460

Pour tout renseignement concernant cette annexe : voir Journal Officiel de l'Office européen des brevets, No.12/82

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- US 5362486 A
- FR 2833489
- WO 9749436 A
- US 2748050 A
- US 3527224 A
- US 3591767 A
- US 3667472 A
- US 3995641 A
- US 4035334 A
- US 4650826 A
- FR 2679771
- EP 1184426 A
- US 6225198 B
- US 5990479 A
- US 4578266 A
- US 6224622 B


### Littérature non-brevet citée dans la description


- **PR WELLS.** *Prog. Phys. Org. Chem,* 1968, vol. 6, 111
- **SAYYAH.** *J. Polymer Research,* 2000, 97
- **HOPFF.** *Makromoleculare Chemie,* 1961, 95
- **DE KEYSER.** *J. Pharm. Sci,* 1991, 67
- **KLEMARCZYK.** *Polymer,* 1998, 173
- **BRETON.** *Biomaterials,* 1998, 271
- **COUVREUR.** *Pharmaceutical Research,* 1994, 1270
- **GIPSTEIN.** *J.Org.Chem,* 1980, 1486
- **BACHRACH.** *European Polymer Journal,* 1976, 563
- **WANATABE.** *J.Polymer Science : Part A :Polymer chemistry,* 1994, 2073
- **DABBOUSSI B.O. et al.** CdSe)ZnS core-shell quantum dots : synthesis and characterisation of a size series of highly luminescent nanocristallites. *Journal of physical chemistry B,* 1997, vol. 101, 9463-9475
- **PENG, XIAOGANG et al.** Epitaxial Growth of highly Luminescent CdSe/CdS core/shell nanocrystals with photostability and electronic accessibility. *Journal of the American Chemical Society,* vol. 119 (30), 7019-7029